# EUROPEAN PATENT SPECIFICATION

(11) **EP 4 402 157 B1**
(45) Date of publication and mention of the grant of the patent: **17.12.2025**
(21) Application number: 22801643.2
(22) Date of filing: 14.09.2022
(51) Int. Cl.: C07K 14/52, C07K 7/06, A61P 35/00, A61K 38/00, C07K 19/00

(54) **CLEAVABLE LINKERS**
SPALTBARE LINKER
LIEURS CLIVABLES

(30) Priority: 14.09.2021 US 202163243843 P
(43) Date of publication of application: 24.07.2024
(60) Divisional application: 25214057.9
(73) Proprietor: Xilio Development, Inc., Waltham, Massachusetts 02451 (US)
(72) Inventor: TOMAR, Dheeraj Singh, Bridgewater, Massachusetts 02379 (US); PEDERZOLI-RIBEIL, Magali, Cambridge, Massachusetts 02140 (US); KNUDSEN, Giselle Marcelline, San Anselmo, California 94960 (US)
(74) Representative: CMS Cameron McKenna Nabarro Olswang LLP
(86) International application number: PCT/US2022/076395
(87) International publication number: WO 2023/044321

(56) References cited:
- WO-A1-2020/069398
- WO-A2-02/20715
- US-A1- 2021 130 430
- ANONYMOUS: "Transposase - Lyngbya aestuarii BL J | UniProtKB | UniProt", 22 January 2014 (2014-01-22), XP093004147, Retrieved from the Internet <URL:https://www.uniprot.org/uniprotkb/U7QND7/entry> [retrieved on 20221201]

## Description

### BACKGROUND

Cleavable linkers are a conjugation tool for connecting components together, which can then be cleaved upon exposure to a reagent, to release said conjugated components. These types of linkers are a powerful tool used in chemical biology and the pharmaceutical field. In particular, cleavable linkers can be used in drugs and prodrugs for delivering therapeutic agents to a target of interest, for example to a tumor cell environment.

Cancer is the second leading cause of death in the United States, accounting for more deaths than the next five leading causes (chronic respiratory disease, stroke, accidents, Alzheimer's disease and diabetes). While great strides have been made especially with targeted therapies, there remains a great deal of work to do in this space. Immunotherapy and a branch of this field, immuno-oncology, is creating viable and exciting therapeutic options for treating malignancies. Specifically, it is now recognized that one hallmark of cancer is immune evasion and significant efforts have identified targets and developed therapies to these targets to reactivate the immune system to recognize and treat cancer.

Cytokine therapy is an effective strategy for stimulating the immune system to induce anti-tumor cytotoxicity. In particular, aldesleukin, a recombinant form of interleukin-2 (IL-2), has been approved by the FDA for the treatment of metastatic renal cell carcinoma and melanoma. Unfortunately, cytokines that are administered to patients generally have a very short half-life, thereby requiring frequent dosing. For instance, the product label of aldesleukin, marketed under the brand name Proleukin, states that the drug was shown to have a half-life of 85 minutes in patients who received a 5-minute intravenous (IV) infusion. In addition, administration of high doses of cytokine can cause adverse health outcomes, such as vascular leakage, through systemic immune activation. These findings illustrate the need for developing therapeutics, such as cytokine therapeutics, that effectively target tumors without the side effects associated with systemic immune activation.

Prodrugs in which a cytokine therapeutic is masked by a masking moiety and in which the therapeutic is only active after cleavage of the masking moiety in the tumor cell environment are one way envisaged for addressing this need.

Transposase - *Lyngbya aestuarii* BL J, UniProtKB, UniProt, 22 January 2014 (URL:https://www.uniprot.org/uniprotkb/U7QND7/entry) relates to a 37-mer polypeptide sequence. US2021130430 features fusion proteins that are conditionally active variants of a cytokine of interest. WO2020069398 provides cytokines or functional fragments thereof that, in some embodiments, are engineered to be masked by a masking moiety at one or more receptor binding site(s) of the cytokine or functional fragment thereof. WO0220715 relates to pharmaceutical compositions comprising a targeting peptide, a protease specific cleavable peptide, and a chemotherapeutic drug that when conjugated are substantially inactive, but upon degradation of the cleavable sequence by a proteolytic enzyme abundant in or within that target cancer cell, the chemotherapeutic drug is released and becomes active, and to the use of these compositions for treatment of cancer.

### SUMMARY

The invention is as defined in the claims. The following aspects are useful for understanding the invention but are not part of the claimed invention in their entirety. The reference to methods of treatment by therapy or surgery or *in vivo* diagnosis methods in the examples of this description are to be interpreted as references to compounds, pharmaceutical compositions and medicaments of the present invention for use in those methods.

This invention provides novel proteolytically cleavable peptide linkers comprising proteolytically cleavable peptides in polypeptides. Said polypeptide is an engineered polypeptide construct, comprising two components joined together via a proteolytically cleavable peptide linker of the invention.

The proteolytically cleavable peptide acts as a substrate for protease(s) (e.g., MMPs and/or cathepsin B). The proteolytically cleavable peptide linker is positioned within the polypeptide so that the linker cleaves by protease action in a protease rich environment, such as a tumor cell environment. Upon proteolytic cleavage, the polypeptide separates to form cleavage products. This invention also relates to cleavage products of said polypeptides, and methods related to the use of the same.

Provided herein is a polypeptide comprising a proteolytically cleavable peptide linker, wherein the linker comprises a proteolytically cleavable peptide (CP) consisting of the amino acid sequence PVSLRSGS (SEQ ID NO: 1) or disclosed herein GMPKDLYHAS (SEQ ID NO. 2), or RPLALWRS (SEQ ID NO: 3), or TQKPLGLS (SEQ ID NO:4), or APAGLIVPYN (SEQ ID NO:5), or PANLVAPDP (SEQ ID NO: 6), or IVGRPRHQGV (SEQ ID NO:7), or RSKYLATA(SEQ ID NO:8).

Also provided herein is a polypeptide construct comprising a proteolytically cleavable peptide linker, wherein the linker comprises a proteolytically cleavable peptide (CP) consisting of the amino acid sequence PVSLRSGS (SEQ ID NO: 1), or disclosed herein GMPKDLYHAS (SEQ ID NO: 2), or RPLALWRS (SEQ ID NO: 3), or TQKPLGLS (SEQ ID NO: 4), or APAGLIVPYN (SEQ ID NO:5), or PANLVAPDP (SEQ ID NO: 6), or IVGRPRHQGV(SEQ ID NO:7), or RSKYLATA(SEQ ID NO:8).

The proteolytically cleavable peptide (CP) is PVSLRSGS (SEQ ID NO:1).

In some embodiments, not according to the claimed invention, the proteolytically cleavable peptide (CP) is GMPKDLYHAS (SEQ ID NO:2).

In some embodiments, not according to the claimed invention, the proteolytically cleavable peptide (CP) is RPLALWRS (SEQ ID NO:3).

In some embodiments, not according to the claimed invention, the proteolytically cleavable peptide (CP) is TQKPLGLS (SEQ ID NO:4).

In some embodiments, not according to the claimed invention, the proteolytically cleavable peptide (CP) is APAGLIVPYN (SEQ ID NO:5).

In some embodiments, not according to the claimed invention, the proteolytically cleavable peptide (CP) is PANLVAPDP (SEQ ID NO:6).

In some embodiments, not according to the claimed invention, the proteolytically cleavable peptide (CP) is IVGRPRHQGV (SEQ ID NO:7).

In some embodiments, not according to the claimed invention, the proteolytically cleavable peptide (CP) is RSKYLATA (SEQ ID NO:8).

In some embodiments, the proteolytically cleavable peptide linker is from 8 to 25 amino acids in length.

In some embodiments, the proteolytically cleavable peptide linker is from 10 to 25 amino acids in length.

In some embodiments, the proteolytically cleavable peptide linker is 8, 9, 10, 11, 12, 13, 14, 15, 16, 17, 18, 19, 20, 21, 22, 23, 24 or 25 amino acids in length.

In some embodiments, disclosed herein, the proteolytically cleavable peptide linker comprises a motif PXXL (SEQ ID NO:11) (X can be any amino acid residue). As a non-limiting example, the proteolytically cleavable peptide linker comprises a substrate sequence of PLGL (SEQ ID NO: 12). In some embodiments, the substrate sequence comprises PLAL (SEQ ID NO:13), PAGL (SEQ ID NO:14), PVSL (SEQ ID NO:15), or PANL (SEQ ID NO:16).

In some embodiments, the polypeptide construct comprises more than one proteolytically cleavable peptide linker.

The polypeptide construct comprises a therapeutic moiety.

The therapeutic moiety is a cytokine moiety.

In some embodiments, the proteolytically cleavable peptide linker is covalently bonded directly to the therapeutic moiety.

In some embodiments, the proteolytically cleavable peptide linker is located within the construct between the therapeutic moiety and a carrier moiety.

In some embodiments, the proteolytically cleavable peptide linker is located within the carrier moiety.

In some embodiments, not according to the claimed invention, the polypeptide construct comprises a single polypeptide chain.

The polypeptide construct comprises more than one polypeptide chain.

In some embodiments, the proteolytically cleavable peptide linker is present in the same polypeptide chain as the therapeutic moiety.

In some embodiments, the proteolytically cleavable peptide linker is present in a different polypeptide chain to the therapeutic moiety.

In some embodiments, the polypeptide construct is a prodrug.

The polypeptide or polypeptide construct further comprises a masking moiety.

In some embodiments, not according to the claimed invention, the masking moiety is present in the same polypeptide chain as the therapeutic moiety.

The masking moiety is present in a different polypeptide chain to the therapeutic moiety.

The polypeptide construct comprises a half-life extension moiety.

In some embodiments, the polynucleotide construct comprises a targeting moiety.

Further provided herein is a cytokine prodrug as described herein, wherein the cytokine prodrug comprises a masking moiety and the masking moiety comprises a domain of the extracellular domain of the cytokine receptor.

Provided herein is a masked cytokine comprising:
a first polypeptide chain comprising a masking moiety linked to a first half-life extension moiety via a first linker; and
a second polypeptide chain comprising a cytokine moiety thereof linked to a second half-life extension moiety via a second linker,
wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO: 1), or disclosed herein GMPKDLYHAS (SEQ ID NO:2), or RPLALWRS (SEQ ID NO:3), or TQKPLGLS (SEQ ID NO: 4), or APAGLIVPYN (SEQ ID NO:5), or PANLVAPDP (SEQ ID NO:6), or IVGRPRHQGV (SEQ ID NO:7), or RSKYLATA (SEQ ID NO:8).

In some embodiments, the first proteolytically cleavable peptide linker and the second proteolytically cleavable peptide linker comprise the same amino acid sequence. In other embodiments, the first proteolytically cleavable peptide linker and the second proteolytically cleavable peptide linker comprise different amino acid sequences.

In some embodiments, the first polypeptide chain comprises:

**N' HL1-L1-MM** C'

and the second polypeptide chain comprises:

   **N' HL2-L2-C** C'
where HL1 is the first half life extension domain, L1 is the first linker, MM is the masking moiety, HL2 is the second half life extension domain, L2 is the second linker, and C is the cytokine moiety,
wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO: 1) or disclosed herein GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO: 6), or IVGRPRHQGV (SEQ ID NO: 7), or RSKYLATA (SEQ ID NO:8).

Also provided herein but not according to the claimed invention, is a masked cytokine comprising a polypeptide chain comprising formula:

**N' HL-L2-C-L1-MM** C'

where HL is the half-life extension domain, L1 is the first linker, MM is the masking moiety, L2 is the second linker, and C is the cytokine moiety, wherein at least the first linker comprises a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2) , or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).

Also provided herein but not according to the claimed invention, is a masked cytokine comprising a polypeptide chain comprising formula:

N' **HL-L2-MM-L1-C** C'

where HL is the half-life extension domain, L1 is the first linker, MM is the masking moiety, L2 is the second linker, and C is the cytokine moiety thereof, wherein at least the first linker comprises a proteolytically cleavable peptide linker comprising a proteolytically cleavable peptide (CP) consisting of the amino acid sequence PVSLRSGS (SEQ ID NO:1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).

Also provided herein is a targeted cytokine comprising: a targeting moiety; a cytokine or a fragment thereof; a masking moiety; and a half-life extension domain comprising a first Fc polypeptide linked to the cytokine or a fragment thereof through a first linker and a second Fc polypeptide linked to the masking moiety through a second linker, and wherein the targeting moiety is linked to the Fc domain through one or both of the first and second Fc polypeptides. In this targeted cytokine construct, the first or the second linker is a cleavable linker such that the masking moiety releases the cytokine or a fragment thereof upon cleavage. The first cleavable linker or the second cleavable linker comprises a proteolytically cleavable peptide linker comprising a proteolytically cleavable peptide (CP) consisting of the amino acid sequence PVSLRSGS (SEQ ID NO: 1) or disclosed herein GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8).

In some embodiments, the second linker is the proteolytically cleavable linker and the first linker is a non-cleavable linker.

In some embodiments, the first linker is the proteolytically cleavable linker and the second linker is a non-cleavable linker.

In some embodiments, the proteolytically cleavable peptide linker is from 9 to 25 amino acids in length.

The proteolytically cleavable peptide (CP) consists of the amino acid sequence PVSLRSGS (SEQ ID NO:1).

In some embodiments, not according to the present invention, the proteolytically cleavable peptide (CP) consists of the amino acid sequence GMPKDLYHAS (SEQ ID NO:2).

In some embodiments, not according to the present invention, the proteolytically cleavable peptide (CP) consists of the amino acid sequence of RPLALWRS (SEQ ID NO:3).

In some embodiments, not according to the present invention, the proteolytically cleavable peptide (CP) consists of the amino acid sequence of TQKPLGLS (SEQ ID NO:4).

In some embodiments, not according to the present invention, the proteolytically cleavable peptide (CP) consists of the amino acid sequence of APAGLIVPYN (SEQ ID NO:5).

In some embodiments, not according to the present invention, the proteolytically cleavable peptide (CP) consists of the amino acid sequence of PANLVAPDP (SEQ ID NO:6).

In some embodiments, not according to the present invention, the proteolytically cleavable peptide (CP) consists of the amino acid sequence of IVGRPRHQGV (SEQ ID NO:7).

In some embodiments, not according to the present invention, the proteolytically cleavable peptide (CP) consists of the amino acid sequence of RSKYLATA (SEQ ID NO:8).

In some embodiments, the cytokine moiety comprises a wild-type cytokine moiety or variant cytokine moiety.

In some embodiments, the cytokine moiety is an IL-2 cytokine moiety.

In some embodiments, the IL-2 cytokine moiety comprises an IL-2 cytokine or fragment thereof.

In some embodiments, the cytokine moiety is an IL-12 cytokine moiety.

In some embodiments, the IL-12 cytokine moiety comprises an IL-12 cytokine or fragment thereof.

In some embodiments, the cytokine moiety is an IL-15 cytokine moiety.

In some embodiments, the IL-15 cytokine moiety comprises an IL-15 cytokine or fragment thereof.

In some embodiments, the masked cytokine further comprises a domain comprising an IL-15Rα subunit or a functional fragment thereof ('IL-15Rα domain').

In some embodiments, the masking moiety comprises a domain of the extracellular domain of the cytokine receptor.

In some embodiments, the half-life extension domains are each an Fc domain or a fragment thereof.

In some embodiments, the targeting moiety is an antibody or antigen binding fragment thereof.

Provided herein is a cleavage product preparable by proteolytic cleavage of the proteolytically cleavable linker in a polypeptide or polypeptide construct, a cytokine prodrug, a masked cytokine, or a targeted cytokine as described anywhere herein.

Provided herein is a nucleic acid encoding a polypeptide construct as described anywhere herein.

Provided herein is a nucleic acid encoding a cytokine prodrug as described anywhere herein.

Provided herein is a nucleic acid encoding a masked cytokine as described anywhere herein.

Provided herein is a nucleic acid encoding a targeted cytokine as described anywhere herein.

Provided herein is a nucleic acid encoding a cleavage product as described anywhere herein.

Provided herein is a vector comprising a nucleic acid as described anywhere herein.

Provided herein is a host cell comprising a nucleic acid as described anywhere herein.

Provided herein is a host cell as described anywhere herein, wherein the host cell is a HEK cell. In some embodiments, the host cell is a CHO cell.

Provided herein is a composition comprising the polypeptide construct as described anywhere herein.

Provided herein is a composition comprising a cytokine prodrug as described anywhere herein.

Provided herein is a composition comprising a masked cytokine as described anywhere herein.

Provided herein is a composition comprising a targeted cytokine as described anywhere herein.

Provided herein is a pharmaceutical composition comprising a polypeptide construct as described anywhere herein and a pharmaceutically acceptable carrier.

Provided herein is a pharmaceutical composition comprising a cytokine prodrug as described anywhere herein, and a pharmaceutically acceptable carrier.

Provided herein is a pharmaceutical composition comprising a masked cytokine as described anywhere herein, and a pharmaceutically acceptable carrier.

Provided herein is a pharmaceutical composition comprising a targeted cytokine as described anywhere herein, and a pharmaceutically acceptable carrier.

Provided herein is a kit comprising a polypeptide construct, or a cytokine prodrug, or a masked cytokine, or a targeted cytokine, or the composition, or the pharmaceutical composition, as described anywhere herein.

Provided herein is a method of producing a masked cytokine as described anywhere herein, comprising culturing the host cell as described herein under a condition that produces the masked cytokine.

Provided herein is a method of producing a targeted cytokine as described anywhere herein, comprising culturing the host cell as described herein under a condition that produces the masked cytokine.

Provided herein is a composition comprising a cleavage product as described anywhere herein.

Provided herein is a pharmaceutical composition comprising the cleavage product as described anywhere herein, and a pharmaceutically acceptable carrier.

Provided herein is a polypeptide construct as described anywhere herein for use in medicine.

Provided herein is a cytokine prodrug as described anywhere herein for use in medicine.

Provided herein is a masked cytokine as described anywhere herein for use in medicine.

Provided herein is a targeted cytokine as described anywhere herein for use in medicine.

Provided herein is a composition as described anywhere herein for use in medicine.

Provided herein is a pharmaceutical composition as described anywhere herein for use in medicine.

Provided herein is a cleavage product as described anywhere herein for use in medicine.

Provided herein is a method of treating or preventing cancer in a subject, the method comprising administering to the subject an effective amount of a polypeptide construct as described anywhere herein. Said methods do not form part of the invention.

Provided herein is a method of treating or preventing cancer in a subject, the method comprising administering to the subject an effective amount of a cytokine prodrug as described anywhere herein. Said methods do not form part of the invention.

Provided herein is a method of treating or preventing cancer in a subject, the method comprising administering to the subject an effective amount of a masked cytokine as described anywhere herein. Said methods do not form part of the invention.

Provided herein is a method of treating or preventing cancer in a subject, the method comprising administering to the subject an effective amount of a targeted cytokine as described anywhere herein. Said methods do not form part of the invention.

Provided herein is a method of treating or preventing cancer in a subject, the method comprising administering to the subject an effective amount of a composition as described anywhere herein. Said methods do not form part of the invention.

Provided herein is a method of treating or preventing cancer in a subject, the method comprising administering to the subject an effective amount of a pharmaceutical composition as described anywhere herein. Said methods do not form part of the invention.

Provided herein is a method of treating or preventing cancer in a subject, the method comprising administering to the subject an effective amount of a cleavage product as described anywhere herein. Said methods do not form part of the invention.

Provided herein is a method of treating or preventing cancer in a subject, the method comprising administering to the subject an effective amount of a cytokine prodrug, or a masked cytokine or a targeted cytokine as described anywhere herein, whereby the cytokine prodrug or masked cytokine, or targeted cytokine is proteolytically cleaved in vivo to produce a cleavage product as described anywhere herein. Said methods do not form part of the invention.

Provided herein is a method of treating or preventing cancer in a subject, the method comprising a step of producing a cleavage product in vivo that is capable of binding to its cognate receptor, where the cleavage product is as described anywhere herein. Said methods do not form part of the invention.

In some embodiments, the cancer is a solid tumor.

Provided herein is a polypeptide construct as described anywhere herein for use in treating or preventing cancer.

Provided herein is a cytokine prodrug as described anywhere herein for use in treating or preventing cancer.

Provided herein is a masked cytokine as described anywhere herein for use in treating or preventing cancer.

Provided herein is a targeted cytokine as described anywhere herein for use in treating or preventing cancer.

Provided herein is a composition as described anywhere herein for use in treating or preventing cancer.

Provided herein is a pharmaceutical composition as described anywhere herein for use in treating or preventing cancer.

Provided herein is a cleavage product as described anywhere herein for use in treating or preventing cancer.

Provided herein is a cleavage product as described anywhere herein for use in a method of treating or preventing cancer, the method comprising a step of a polypeptide drug construct, a cytokine prodrug, or a masked cytokine, or a targeted cytokine as described anywhere herein to a patient, thereby producing the cleavage product by proteolytic cleavage of the proteolytically cleavable peptide linker in vivo.

Provided herein is a cleavage product as described anywhere herein for use in a method of treating or preventing cancer in a subject, the method comprising a step of producing the cleavage product by in vivo proteolytic cleavage of a polypeptide drug construct, a cytokine prodrug, or a masked cytokine, or a targeted cytokine as described anywhere herein, that has been administered to the subject.

### BRIEF DESCRIPTION OF THE DRAWINGS

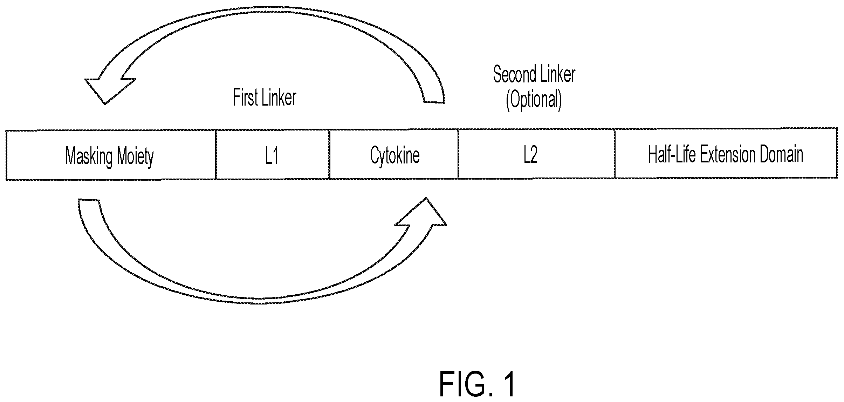

FIG. 1 shows the structure of exemplary embodiments of a masked cytokine, which are not encompassed by the wording of the claims, that includes a masking moiety, a cytokine or functional fragment thereof ("cytokine"), a half-life extension moiety, and a first linker. These exemplary embodiments, which are not encompassed by the wording of the claims, also include a second linker. The first or second linker may be a cleavable linker. As shown by the arrows, while the exemplary embodiments, which are not encompassed by the wording of the claims, shows the masking moiety linked to the first linker, and the cytokine or functional fragment thereof is linked to the first linker and the second linker, the masking moiety and the cytokine or functional fragment thereof can be interchanged such that the cytokine or functional fragment thereof is linked to the first linker, and the masking moiety is linked to the first linker and the second linker. FIG. 1 shows the structure of an exemplary embodiment, which is not encompassed by the wording of the claims, of a masked cytokine as a linear monomer.

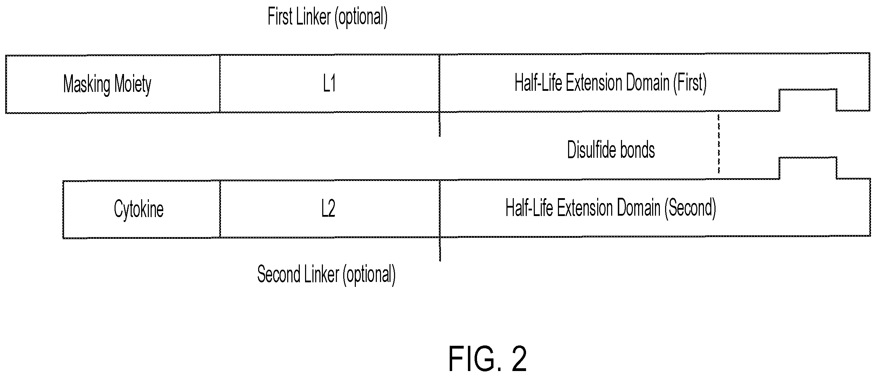

FIG. 2 shows the structure of an exemplary embodiment of a masked cytokine that includes a masking moiety, a cytokine or functional fragment thereof ("cytokine"), a first half-life extension moiety, and a second half-life extension moiety. The exemplary embodiment shown in FIG. 2 also includes a first linker, and a second linker. The first or second linker may be a cleavable linker. The exemplary first and second half-life extension moieties include "knobs into holes" modifications that promote the association of the first half-life extension moiety with the second half-life extension moiety, as shown by the "hole" in the first half-life extension moiety and the "knob" in the second half-life extension moiety. The first half-life extension moiety and the second half-life extension moiety are also shown as associating, at least in part, due to the formation of disulfide bonds. It is to be understood that although the "hole" is depicted as part of the first half-life extension moiety (linked to the masking moiety) and the "knob" is depicted as part of the second half-life extension moiety (linked to the cytokine), the "hole" and the "knob" can alternatively be included in the second half-life extension moiety and the first half-life extension moiety, respectively, so that the "hole" is a part of the second half-life extension moiety (linked to the cytokine) and the "knob" is part of the first half-life extension moiety (linked to masking moiety).
FIG. 3A is a representative diagram of the "heterodimeric" masked cytokine (Structure A) wherein the proteolytically cleavable peptide linker is positioned between the half-life extension moiety and the masking moiety.
FIG. 3B is a representative diagram of the "heterodimeric" masked cytokine (Structure B) wherein the proteolytically cleavable peptide linker is positioned between the half-life extension moiety and the cytokine moiety.
FIG. 3C is a representative diagram of a "heterodimeric" targeted cytokine (Structure C), wherein an antibody is linked to a cytokine moiety and a masking moiety. The proteolytically cleavable peptide linker is positioned on the masking moiety arm.
FIG. 3D is a representative diagram of a "heterodimeric" targeted masked cytokine (Structure D), wherein an antibody is linked to a cytokine moiety and a masking moiety. The proteolytically cleavable peptide linker is positioned on the masking moiety arm.

### DETAILED DESCRIPTION

### 1. POLYPEPTIDE CONSTRUCTS

This disclosure provides novel proteolytically cleavable peptide linkers.

The proteolytically cleavable peptide linker is positioned within a polypeptide, in particular a polypeptide construct so that when the linker cleaves by protease action, the polypeptide construct separates. Thus, the invention relates to a polypeptide construct comprising a proteolytically cleavable peptide linker as described in the claims.

This invention also relates to cleavage products of said polypeptide constructs.

Protease substrate amino acid sequences PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8) have been found to demonstrate very specific cleavage. SEQ ID NO: 1 advantageously is used in the proteolytically cleavable peptide linkers of the invention. In particular, these proteolytically cleavable peptides have been found to demonstrate very specific cleavage in the tumor cell environment compared to non-tumor cell environment. Thus, these proteolytically cleavable peptides advantageously demonstrate tumor-specific cleavage.

A polypeptide construct of the disclosure may comprise any number and/or type of component. The proteolytically cleavable peptide linkers of the invention may be used to conjugate together said components in a polypeptide construct. In some embodiments, the proteolytically cleavable peptide linker may be used to conjugate one group of components to another group of components. In some embodiments, said group of components comprises 1, 2, 3, 4, or 5 or more components.

In some embodiments, it may be advantageous for certain components in the polypeptide construct to separate and for other components to remain linked. For example, in some embodiments, the proteolytically cleavable peptide linker may be used to conjugate a targeting moiety to a therapeutic moiety, where it is desirable for the therapeutic moiety to separate from the targeting moiety, such as once the polypeptide construct has reached a target environment.

### 2. PROTEOLYTICALLY CLEAVABLE PEPTIDE LINKERS

The proteolytically cleavable peptide linkers described herein comprise a proteolytically cleavable peptide (CP) consisting of the amino acid sequence PVSLRSGS (SEQ ID NO:1) or disclosed herein GMPKDLYHAS (SEQ ID NO:2) , or RPLALWRS 9 SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7) , or RSKYLATA (SEQ ID NO:8).

A first aspect of the invention relates to the protease substrate amino acid sequence PVSLRSGS (SEQ ID NO:1).

The proteolytically cleavable peptide (CP) consists of the amino acid sequence PVSLRSGS (SEQ ID NO:1).

In another embodiment of the first aspect, the proteolytically cleavable peptide linker comprises a proteolytically cleavable peptide (CP) consisting of the amino acid sequence PVSLRSGS (SEQ ID NO:1), and is from 8 to 25, 9 to 25, 10 to 25, 10 to 20, 12 to 20, or 12 to 18 amino acids in length. In yet another embodiment of the first aspect which is not encompassed by the wording of the claims, the proteolytically cleavable peptide linker comprises a proteolytically cleavable peptide (CP) consisting of the sequence of PVSLRSGS (SEQ ID NO:1) with less than 4, or less than 3, or less than 2 amino acid substitutions.

A second aspect of the disclosure not encompassed by the wording of the claims, relates to the protease substrate amino acid sequence GMPKDLYHAS (SEQ ID NO:2).

In one embodiment of the second aspect which is not encompassed by the wording of the claims, the proteolytically cleavable peptide (CP) consists of the amino acid sequence GMPKDLYHAS (SEQ ID NO:2).

In another embodiment of the second aspect which is not encompassed by the wording of the claims, the proteolytically cleavable peptide linker comprises a proteolytically cleavable peptide (CP) consisting of the amino acid sequence GMPKDLYHAS (SEQ ID NO:2), and is from 10 to 25, 11 to 25, 12 to 25, 10 to 20, 12 to 20, or 12 to 18 amino acids in length. In yet another embodiment of the second aspect which is not encompassed by the wording of the claims, the proteolytically cleavable peptide linker comprises a proteolytically cleavable peptide (CP) consisting of the sequence of GMPKDLYHAS (SEQ ID NO:2) with less than 5, or less than 4, or less than 3, or less than 2 amino acid substitutions.

A third aspect which is not encompassed by the wording of the claims, relates to the protease substrate amino acid sequence RPLALWRS (SEQ ID NO:3).

In one embodiment of the third aspect which is not encompassed by the wording of the claims, the proteolytically cleavable peptide (CP) consists of the amino acid sequence RPLALWRS (SEQ ID NO:3).

In another embodiment of the third aspect which is not encompassed by the wording of the claims, the proteolytically cleavable peptide linker comprises a proteolytically cleavable peptide (CP) consisting of the amino acid sequence RPLALWRS (SEQ ID NO:3), and is from 8 to 25, 9 to 25, 10 to 25, 10 to 20, 12 to 20, or 12 to 18 amino acids in length. In yet another embodiment of the third aspect which is not encompassed by the wording of the claims, the proteolytically cleavable peptide linker comprises a proteolytically cleavable peptide (CP) consisting of the sequence of RPLALWRS (SEQ ID NO:3) with less than 4, or less than 3, or less than 2 amino acid substitutions.

A fourth aspect which is not encompassed by the wording of the claims, relates to the protease substrate amino acid sequence TQKPLGLS (SEQ ID NO:4).

In one embodiment of the fourth aspect which is not encompassed by the wording of the claims, the proteolytically cleavable peptide (CP) consists of the amino acid sequence TQKPLGLS (SEQ ID NO:4).

In another embodiment of the fourth aspect which is not encompassed by the wording of the claims, the proteolytically cleavable peptide linker comprises a proteolytically cleavable peptide (CP) consisting of the amino acid sequence TQKPLGLS (SEQ ID NO:4), and is from 8 to 25, 9 to 25, 10 to 25, 10 to 20, 12 to 20, or 12 to 18 amino acids in length. In yet another embodiment of the fourth aspect which is not encompassed by the wording of the claims, the proteolytically cleavable peptide linker comprises a proteolytically cleavable peptide (CP) consisting of the sequence of TQKPLGLS (SEQ ID NO:4), with less than 4, or less than 3, or less than 2 amino acid substitutions.

A fifth aspect which is not encompassed by the wording of the claims, relates to the protease substrate amino acid sequence APAGLIVPYN (SEQ ID NO:5).

In one embodiment of the fifth aspect which is not encompassed by the wording of the claims, the proteolytically cleavable peptide (CP) consists of the amino acid sequence APAGLIVPYN (SEQ ID NO:5).

In another embodiment of the fifth aspect which is not encompassed by the wording of the claims, the proteolytically cleavable peptide linker comprises a proteolytically cleavable peptide (CP) consisting of the amino acid sequence APAGLIVPYN (SEQ ID NO:5), and is from 10 to 25, 11 to 25, 12 to 25, 10 to 20, 12 to 20, or 12 to 18 amino acids in length. In yet another embodiment of the fifth aspect which is not encompassed by the wording of the claims, the proteolytically cleavable peptide linker comprises a proteolytically cleavable peptide (CP) consisting of the sequence of APAGLIVPYN (SEQ ID NO:5) with less than 5, less than 4, or less than 3, or less than 2 amino acid substitutions.

A sixth aspect which is not encompassed by the wording of the claims, relates to the protease substrate amino acid sequence PANLVAPDP (SEQ ID NO:6).

In one embodiment of the sixth aspect which is not encompassed by the wording of the claims, the proteolytically cleavable peptide (CP) consists of the amino acid sequence PANLVAPDP (SEQ ID NO:6).

In another embodiment of the sixth aspect which is not encompassed by the wording of the claims, the proteolytically cleavable peptide linker comprises a proteolytically cleavable peptide (CP) consisting of the amino acid sequence PANLVAPDP (SEQ ID NO:6), and is from 9 to 25, 10 to 25, 11 to 25, 12 to 25, 10 to 20, 12 to 20, or 12 to 18 amino acids in length. In yet another embodiment of the sixth aspect which is not encompassed by the wording of the claims, the proteolytically cleavable peptide linker comprises a proteolytically cleavable peptide (CP) consisting of the sequence of PANLVAPDP (SEQ ID NO:6), with less than 4, or less than 3, or less than 2 amino acid substitutions.

A seventh aspect which is not encompassed by the wording of the claims, relates to the protease substrate amino acid sequence IVGRPRHQGV (SEQ ID NO:7).

In one embodiment of the seventh aspect which is not encompassed by the wording of the claims, the proteolytically cleavable peptide (CP) consists of the amino acid sequence IVGRPRHQGV (SEQ ID NO:7).

In another embodiment of the seventh aspect which is not encompassed by the wording of the claims, the proteolytically cleavable peptide linker comprises a proteolytically cleavable peptide (CP) consisting of the amino acid sequence IVGRPRHQGV (SEQ ID NO:7), and is from 10 to 25, 11 to 25, 12 to 25, 10 to 20, 12 to 20, or 12 to 18 amino acids in length. In yet another embodiment of the seventh aspect which is not encompassed by the wording of the claims, the proteolytically cleavable peptide linker comprises a proteolytically cleavable peptide (CP) consisting of the sequence of IVGRPRHQGV (SEQ ID NO:7), with less than 5, or less than 4, or less than 3, or less than 2 amino acid substitutions.

An eighth aspect which is not encompassed by the wording of the claims, relates to the protease substrate amino acid RSKYLATA.

In one embodiment of the eighth aspect which is not encompassed by the wording of the claims, the proteolytically cleavable peptide (CP) consists of the amino acid sequence RSKYLATA (SEQ ID NO:8).

In another embodiment of the eighth aspect which is not encompassed by the wording of the claims, the proteolytically cleavable peptide linker comprises a proteolytically cleavable peptide (CP) consisting of the amino acid sequence RSKYLATA (SEQ ID NO:8), and is from 8 to 25, 9 to 25, 10 to 25, 11 to 25, 12 to 25, 10 to 20, 12 to 20, or 12 to 18 amino acids in length. In yet another embodiment of the eighth aspect which is not encompassed by the wording of the claims, the proteolytically cleavable peptide linker comprises a proteolytically cleavable peptide (CP) consisting of the sequence of RSKYLATA (SEQ ID NO:8), with less than 4, or less than 3, or less than 2 amino acid substitutions.

In some embodiments, the proteolytically cleavable peptide linker is 8, 9, 10, 11, 12, 13, 14, 15, 16, 17, 18, 19, 20, 21, 22, 23, 24 or 25 amino acids in length.

Disclosed herein, in some embodiments, the proteolytically cleavable peptide linker comprises a motif PXXL (SEQ ID NO:11) (X can be any amino acid residue). As a non-limiting example, the proteolytically cleavable peptide linker comprises a substrate sequence of PLGL (SEQ ID NO: 12). In some embodiments, the substrate sequence comprises PLAL (SEQ ID NO: 13), PAGL (SEQ ID NO:14), PVSL (SEQ ID NO: 15), or PANL (SEQ ID NO:16).

Any polypeptide or polypeptide construct described herein comprises a proteolytically cleavable peptide (CP) or proteolytically cleavable linker according to the first aspect of the invention.

Any polypeptide or polypeptide construct described herein may comprise a proteolytically cleavable peptide (CP) or proteolytically cleavable linker according to the second aspect. Said polypeptides or polypeptide constructs do not form part of the claimed invention.

Any polypeptide or polypeptide construct described herein may comprise a proteolytically cleavable peptide (CP) or proteolytically cleavable linker according to the third aspect. Said polypeptides or polypeptide constructs do not form part of the claimed invention.

Any polypeptide or polypeptide construct described herein may comprise a proteolytically cleavable peptide (CP) or proteolytically cleavable linker according to the fourth aspect. Said polypeptides or polypeptide constructs do not form part of the claimed invention.

Any polypeptide or polypeptide construct described herein may comprise a proteolytically cleavable peptide (CP) or proteolytically cleavable linker according to the fifth aspect. Said polypeptides or polypeptide constructs do not form part of the claimed invention.

Any polypeptide or polypeptide construct described herein may comprise a proteolytically cleavable peptide (CP) or proteolytically cleavable linker according to the sixth aspect. Said polypeptides or polypeptide constructs do not form part of the claimed invention.

Any polypeptide or polypeptide construct described herein may comprise a proteolytically cleavable peptide (CP) or proteolytically cleavable linker according to the seventh aspect. Said polypeptides or polypeptide constructs do not form part of the claimed invention.

Any polypeptide or polypeptide construct described herein may comprise a proteolytically cleavable peptide (CP) or proteolytically cleavable linker according to the eighth aspect. Said polypeptides or polypeptide constructs do not form part of the claimed invention.

### 3. POLYPEPTIDE DRUG CONSTRUCTS

The invention also relates to the use of the proteolytically cleavable peptide linkers according to the claimed invention in polypeptide drug constructs for delivering a therapeutic moiety to a target of interest, and the use of the same. Accordingly, provided herein also include polypeptide drug constructs comprising the proteolytically cleavable peptide linkers as described herein.

Due to their tumor specificity, cleavable peptides PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV (SEQ ID NO:7), or RSKYLATA (SEQ ID NO:8) advantageously can be used in proteolytically cleavable peptide linkers in polypeptide drug constructs where the target of the drug is a tumor cell, wherein any systemic side effects of the administered protein therapeutic may be reduced. In some embodiments, cleavable peptides PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6) are cleavable by tumor specific MMPs. In other embodiments, cleavable peptides IVGRPRHQGV (SEQ ID NO:7), or RSKYLATA (SEQ ID NO:8) are cleavable by Cathepsin. For example, a proteolytically cleavable peptide linker comprising the cleavable peptide consisting of RPLALWRS (SEQ ID NO: 3) can be cleaved by matrix metalloproteinase-7 (MMP7).

The proteolytically cleavable peptide linker may be bonded directly or indirectly to the therapeutic moiety within the polypeptide drug construct. Where the polypeptide drug construct comprises more than one polypeptide chain, the proteolytically cleavable peptide linker may be present in the same polypeptide chain as the therapeutic moiety or in a different polypeptide chain.

The part of the construct other than the therapeutic moiety can be considered as a carrier moiety. Where the proteolytically cleavable peptide linker is covalently bonded directly to the therapeutic moiety, the proteolytically cleavable peptide linker will be located within the drug construct between the therapeutic moiety and the carrier moiety. Alternatively, the proteolytically cleavable peptide linker may be located within the carrier moiety such that the molecule that separates away after cleavage comprises the therapeutic moiety and a part of the carrier moiety.

The polypeptide drug construct comprises a masking moiety. In some examples, the proteolytically cleavable peptide linker is covalently bonded directly to the therapeutic moiety and the proteolytically cleavable peptide linker is located within the drug construct between the therapeutic moiety and the masking moiety.

In some embodiments, the polypeptide drug construct comprises a targeting moiety. In some examples, the proteolytically cleavable peptide linker is covalently bonded directly to the therapeutic moiety, and the proteolytically cleavable peptide linker is located within the drug construct between the therapeutic moiety and the targeting moiety.

The polypeptide drug construct comprising one or more proteolytically cleavable peptide linkers may be a prodrug. Where the tumor-specific cleavable linker is used in a prodrug for delivering a therapeutic moiety to a tumor cell environment, the remainder of the molecule from which the therapeutic moiety separates away after cleavage may comprise a masking moiety, which inhibits the biological activity of the therapeutic moiety in the prodrug such that the therapeutic moiety is biologically active only after cleavage of the proteolytically cleavable peptide linker in the tumor cell environment. The drug construct thus may be considered to be a masked drug construct.

The masking moiety may be present in the same polypeptide chain as the therapeutic moiety. Alternatively, the masking moiety may be present in a first polypeptide chain and the therapeutic moiety may be present in a second polypeptide chain. The proteolytically cleavable peptide linker may be present in the first or second polypeptide chain.

By using a masking moiety, the systemic side effects of an administered protein therapeutic can be reduced by interfering with the binding capability of the therapeutic. By masking the therapeutic using a proteolytically cleavable peptide linker, the binding capability that is interfered with by using the masking moiety can be restored by cleavage of the proteolytically cleavable peptide linker at the tumor microenvironment. Thus, the prodrugs provided herein are engineered to precisely target pharmacological activity to the tumor microenvironment by exploiting one of the hallmarks of cancer, high local concentrations of active protease. This feature of the tumor microenvironment is used to transform a systemically inert molecule into a locally active molecules in the form of a cleavage product. Activation of the therapeutic moiety at the tumor microenvironment significantly reduces systemic toxicities that can be associated with drugs that are administered to a subject in active form.

The drug construct provided herein comprises half-life extension moiety. A long half-life in vivo is important for therapeutic proteins. Unfortunately, therapeutics that are administered to a subject can have a short half-life since they are normally cleared rapidly from the subject by mechanisms including clearance by the kidney and endocytic degradation. Thus, in the drug constructs provided herein, a half-life extension moiety may be included for the purpose of extending the half-life of the therapeutic moiety in vivo. The term "half-life extension moiety" encompasses, for example, PEG, albumin, antibodies and antibody fragments. The half-life extension moiety may comprise an antibody or fragment thereof. The half-life extension moiety may comprise an Fc domain or fragment thereof. Where a polypeptide construct comprises a first half-life extension moiety and a second half-life extension moiety, and the first half-life extension moiety is associated with the second half-life extension moiety, both half-life extension moieties may comprise an Fc domain or fragment thereof.

In some embodiments, the polypeptide drug construct provided herein comprises an antibody moiety (e.g., an antibody or an antigen binding fragment thereof). In some embodiments, the polypeptide construct comprises a therapeutic moiety and a masking moiety. In some embodiments, the proteolytically cleavable peptide linker of the present invention is located between the antibody moiety and the masking moiety. In some embodiments, the proteolytically cleavable peptide linker of the present invention is located between the antibody moiety and the cytokine moiety. The antibody specifically binds to a protein, e.g., a tumor specific antigen, thereby carrying the linked therapeutic moiety to the tumor.

### 3.1 Therapeutic moieties

The polypeptide drug constructs or prodrugs described herein may comprise any therapeutic moiety.

Provided herein, in some embodiments, is a cytokine prodrug where the therapeutic moiety is a cytokine moiety. The masking moiety in the cytokine prodrug may comprise a domain of the extracellular domain of the cytokine receptor. The cytokine prodrug thus may be considered to be a masked cytokine.

The cytokine moiety may comprise a wild-type cytokine moiety or variant cytokine moiety.

The cytokine moiety may include but is not limited to IL-1, IL-2, IL-3, IL-4, IL-5, IL-6, IL-7, IL-9, IL-10, IL-11, IL-12, IL-13, IL-14, IL-15, IL-16, IL-17, IL-18, IL-20, IL-22, IL-34, TNF-α, TNF-β, CXCL8 (IL-8), G-CSF, GM-CSF, LIF, OSM, IFN-α, IFN-β, IFN-γ, CD154, LT-β, 4-1BBL, APRIL, CD70, CD153, CD178, GITRL, LIGHT, OX40L, TALL-1, TRAIL, TWEAK, TRANCE, TGF-β, M-CSF, or MSP, or a variant thereof, or a fragment thereof.

Masked cytokines (such as IL-2, IL-12 and IL-15 cytokines) are described in PCT/US2019/053588, PCT/US2021/025103, PCT/US2021/025107 and PCT/US2021/025100.

In some examples, cytokine is IL-15.

In some examples, cytokine is IL-2.

In some examples, cytokine is IL-12.

### 3.2 Cytokine prodrugs

Cytokines play a role in cellular signalling, particularly in cells of the immune system. Provided herein is a cytokine moiety comprising a cytokine (e.g., IL-15 cytokine) or functional fragment thereof for use in a masked cytokine comprising the proteolytically cleavable peptide linker described anywhere herein, or cleavage product thereof.

### 3.2.1 'Heterodimeric' masked cytokines

Provided herein, in some embodiments, is a masked cytokine comprising a masking moiety in a first polypeptide chain and a cytokine moiety thereof in a second polypeptide chain. Such masked cytokines may be referred to as 'heterodimeric' masked cytokines.

In some embodiments, the masked cytokine comprises a protein heterodimer comprising:
a) a first polypeptide chain comprising a masking moiety linked to a first half-life extension moiety via a first linker; and
b) a second polypeptide chain comprising a cytokine moiety thereof linked to a second half-life extension moiety via a second linker,

wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO: 1) or disclosed herein GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8).

In some embodiments, the first linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). The proteolytically cleavable peptide linker may be as described anywhere herein. In some embodiments, the first linker is a proteolytically cleavable peptide linker comprising a proteolytically cleavable peptide (CP) consisting of the amino acid sequence PVSLRSGS (SEQ ID NO: 1). In some embodiments, the first linker is a proteolytically cleavable peptide linker comprising a proteolytically cleavable peptide (CP) consisting of the amino acid sequence GMPKDLYHAS (SEQ ID NO:2). In some embodiments, the first linker is a proteolytically cleavable peptide linker comprising a proteolytically cleavable peptide (CP) consisting of the amino acid sequence RPLALWRS (SEQ ID NO:3). In some embodiments, the first linker is a proteolytically cleavable peptide linker comprising a proteolytically cleavable peptide (CP) consisting of the amino acid sequence TQKPLGLS (SEQ ID NO:4). In some embodiments, the first linker is a proteolytically cleavable peptide linker comprising a proteolytically cleavable peptide (CP) consisting of the amino acid sequence APAGLIVPYN (SEQ ID NO:5). In some embodiments, the first linker is a proteolytically cleavable peptide linker comprising a proteolytically cleavable peptide (CP) consisting of the amino acid sequence PANLVAPDP (SEQ ID NO:6). In some embodiments, the first linker is a proteolytically cleavable peptide linker comprising a proteolytically cleavable peptide (CP) consisting of the amino acid sequence IVGRPRHQGV (SEQ ID NO:7). In some embodiments, the first linker is a proteolytically cleavable peptide linker comprising a proteolytically cleavable peptide (CP) consisting of the amino acid sequence RSKYLATA (SEQ ID NO:8). In some embodiments, the first linker is a proteolytically cleavable peptide linker and the second linker is a non-cleavable linker.

In some embodiments, the second linker is a proteolytically cleavable peptide linker comprising a proteolytically cleavable peptide (CP) consisting of the amino acid sequence PVSLRSGS (SEQ ID NO: 1) or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). The proteolytically cleavable peptide linker may be as described anywhere herein. In some embodiments, the second linker is a proteolytically cleavable peptide linker comprising a proteolytically cleavable peptide (CP) consisting of the amino acid sequence PVSLRSGS (SEQ ID NO: 1). In some embodiments, the second linker is a proteolytically cleavable peptide linker comprising a proteolytically cleavable peptide (CP) consisting of the amino acid sequence GMPKDLYHAS (SEQ ID NO:2). In some embodiments, the second linker is a proteolytically cleavable peptide linker comprising a proteolytically cleavable peptide (CP) consisting of the amino acid sequence RPLALWRS (SEQ ID NO:3). In some embodiments, the second linker is a proteolytically cleavable peptide linker comprising a proteolytically cleavable peptide (CP) consisting of the amino acid sequence TQKPLGLS (SEQ ID NO:4). In some embodiments, the second linker is a proteolytically cleavable peptide linker comprising a proteolytically cleavable peptide (CP) consisting of the amino acid sequence APAGLIVPYN (SEQ ID NO:5). In some embodiments, the second linker is a proteolytically cleavable peptide linker comprising a proteolytically cleavable peptide (CP) consisting of the amino acid sequence PANLVAPDP (SEQ ID NO:6). In some embodiments, the second linker is a proteolytically cleavable peptide linker comprising a proteolytically cleavable peptide (CP) consisting of the amino acid sequence IVGRPRHQGV (SEQ ID NO:7). In some embodiments, the second linker is a proteolytically cleavable peptide linker comprising a proteolytically cleavable peptide (CP) consisting of the amino acid sequence RSKYLATA (SEQ ID NO:8). In some embodiments, the second linker is a proteolytically cleavable peptide linker and the first linker is non-cleavable. The non-cleavable linker may be as described anywhere herein.

The proteolytically cleavable peptide linker may be as described anywhere herein.

In some embodiments, in the first polypeptide chain, the first half life extension domain is linked to the amino terminus of the first linker and the carboxy terminus of the first linker is linked to the amino terminus of the masking moiety and, in the second polypeptide chain, the second half life extension domain is linked to the amino terminus of the second linker and the carboxy terminus of the second linker is linked to the amino terminus of the cytokine moiety thereof.

In some embodiments, the first polypeptide chain comprises:

**N' HL1-L1-MM** C'

and the second polypeptide chain comprises:

**N' HL2-L2-C** C'

where HL1 is the first half life extension domain, L1 is the first linker, MM is the masking moiety, HL2 is the second half life extension domain, L2 is the second linker, and C is the cytokine moiety thereof .

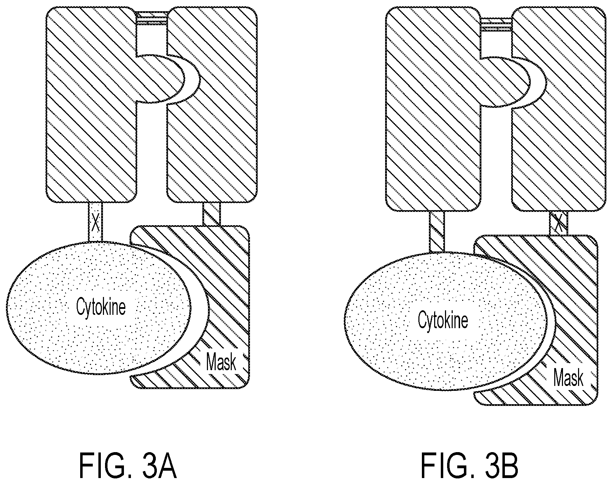

In some embodiments, the second linker is the proteolytically cleavable linker and the first linker is a non-cleavable linker. This arrangement is described herein as 'Structure A' (FIG. 3A). In some embodiments, the first polypeptide chain comprises:

**N' HL1-non-cleavable L1-MM** C'

and the second polypeptide chain comprises:

**N' HL2-cleavable L2-C** C'

In some embodiments, the first linker is the proteolytically cleavable linker and the second is a non-cleavable linker. This arrangement is described herein as 'Structure B' (FIG. 3B). In some embodiments, the first polypeptide chain comprises:

N' **HL1- cleavable L1-MM** C'

and the second polypeptide chain comprises:

N' **HL2- non-cleavable L2-C** C'

### 3.2.2 'Linear' masked cytokines

Provided herein, in some embodiments not according to the claimed invention, is a masked cytokine comprising a masking moiety and a cytokine moiety thereof linked in a single polypeptide chain.

In some embodiments not according to the claimed invention, the masked cytokine comprises a polypeptide chain comprising formula:

**N' HL-L2-C-L1-MM** C'

where HL is the half-life extension domain, L1 is the first linker, MM is the masking moiety, L2 is the second linker, and C is the cytokine moiety thereof, wherein at least the first linker comprises a proteolytically cleavable peptide as described anywhere herein.

In some embodiments not according to the claimed invention, the masked cytokine comprises a polypeptide chain comprising formula:

**N' HL-L2-MM-L1-C** C'

where HL is the half-life extension domain, L1 is the first linker, MM is the masking moiety, L2 is the second linker, and C is the cytokine moiety thereof, wherein at least the first linker comprises a proteolytically cleavable peptide as described anywhere herein.

In some embodiments, the first linker is a cleavable linker as described anywhere herein. In some embodiments, the second linker is a non-cleavable linker.

### 3.2.3 'Targeted' cytokine

Provided herein, in some embodiments, is a masked cytokine comprising a targeting moiety, a masking moiety and a cytokine moiety and a half-life extension moiety (e.g., Fc domain). The Fc domain comprising a first Fc polypeptide linked to the cytokine moiety through a first linker and a second Fc polypeptide linked to the masking moiety through a second linker. The targeting moiety (TM) (e.g., an antibody or antigen binding domain thereof) is linked to the Fc domain through one or both of the first and second Fc polypeptides. In some embodiments, the targeting moiety comprises half-life extension properties. The first or the second linker is a cleavable linker such that the masking moiety releases the cytokine or a fragment thereof upon cleavage.

In some embodiments, the second linker between the second Fc polypeptide and the masking moiety is a cleavable linker and the first linker is a non-cleavable linker. In some embodiments, the second linker is a proteolytically cleavable peptide linker comprising a proteolytically cleavable peptide (CP) consisting of the amino acid sequence PVSLRSGS (SEQ ID NO:1), or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). The proteolytically cleavable peptide linker may be as described anywhere herein. In some embodiments, the second linker is a proteolytically cleavable peptide linker comprising a proteolytically cleavable peptide (CP) consisting of the amino acid sequence PVSLRSGS (SEQ ID NO: 1). In some embodiments, the second linker is a proteolytically cleavable peptide linker comprising a proteolytically cleavable peptide (CP) consisting of the amino acid sequence GMPKDLYHAS (SEQ ID NO:2). In some embodiments, the second linker is a proteolytically cleavable peptide linker comprising a proteolytically cleavable peptide (CP) consisting of the amino acid sequence RPLALWRS (SEQ ID NO:3). In some embodiments, the second linker is a proteolytically cleavable peptide linker comprising a proteolytically cleavable peptide (CP) consisting of the amino acid sequence TQKPLGLS (SEQ ID NO:4). In some embodiments, the second linker is a proteolytically cleavable peptide linker comprising a proteolytically cleavable peptide (CP) consisting of the amino acid sequence APAGLIVPYN (SEQ ID NO:5). In some embodiments, the second linker is a proteolytically cleavable peptide linker comprising a proteolytically cleavable peptide (CP) consisting of the amino acid sequence PANLVAPDP (SEQ ID NO:6). In some embodiments, the second linker is a proteolytically cleavable peptide linker comprising a proteolytically cleavable peptide (CP) consisting of the amino acid sequence IVGRPRHQGV (SEQ ID NO:7). In some embodiments, the second linker is a proteolytically cleavable peptide linker comprising a proteolytically cleavable peptide (CP) consisting of the amino acid sequence RSKYLATA (SEQ ID NO:8).

In some embodiments, the first linker between the first Fc polypeptide and the cytokine moiety is a cleavable linker and the second linker is a non-cleavable linker. In some embodiments, the first linker is a proteolytically cleavable peptide linker comprising a proteolytically cleavable peptide (CP) consisting of the amino acid sequence PVSLRSGS (SEQ ID NO:1), or GMPKDLYHAS (SEQ ID NO:2), or RPLALWRS (SEQ ID NO:3), or TQKPLGLS (SEQ ID NO:4), or APAGLIVPYN (SEQ ID NO:5), or PANLVAPDP (SEQ ID NO:6), or IVGRPRHQGV(SEQ ID NO:7), or RSKYLATA (SEQ ID NO:8). In some embodiments, the first linker is a proteolytically cleavable peptide linker comprising a proteolytically cleavable peptide (CP) consisting of the amino acid sequence PVSLRSGS (SEQ ID NO: 1). In some embodiments, the first linker is a proteolytically cleavable peptide linker comprising a proteolytically cleavable peptide (CP) consisting of the amino acid sequence GMPKDLYHAS (SEQ ID NO:2). In some embodiments, the first linker is a proteolytically cleavable peptide linker comprising a proteolytically cleavable peptide (CP) consisting of the amino acid sequence RPLALWRS (SEQ ID NO:3). In some embodiments, the first linker is a proteolytically cleavable peptide linker comprising a proteolytically cleavable peptide (CP) consisting of the amino acid sequence TQKPLGLS (SEQ ID NO:4). In some embodiments, the first linker is a proteolytically cleavable peptide linker comprising a proteolytically cleavable peptide (CP) consisting of the amino acid sequence APAGLIVPYN (SEQ ID NO:5). In some embodiments, the first linker is a proteolytically cleavable peptide linker comprising a proteolytically cleavable peptide (CP) consisting of the amino acid sequence PANLVAPDP (SEQ ID NO:6). In some embodiments, the first linker is a proteolytically cleavable peptide linker comprising a proteolytically cleavable peptide (CP) consisting of the amino acid sequence IVGRPRHQGV (SEQ ID NO:7). In some embodiments, the first linker is a proteolytically cleavable peptide linker comprising a proteolytically cleavable peptide (CP) consisting of the amino acid sequence RSKYLATA (SEQ ID NO:8).

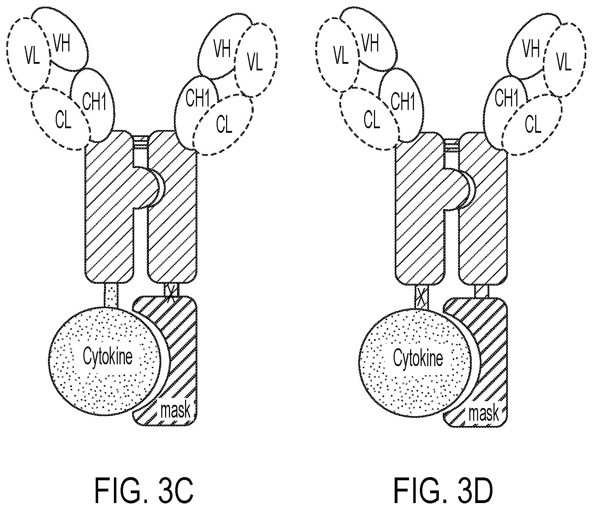

In some embodiments, the targeting moiety (TM) second linker is the proteolytically cleavable linker and the first linker is a non-cleavable linker. This arrangement is described herein as 'Structure C' (FIG. 3C). In some embodiments, the first polypeptide chain comprises:

N' **TM1-non-cleavable L1-MM** C'

and the second polypeptide chain comprises:

N' **TM2-cleavable L2-C** C'

where TM1 and TM2 are the targeting moieties, L1 is the first linker, MM is the masking moiety, L2 is the second linker, and C is the cytokine moiety.

In some embodiments, the first linker is the proteolytically cleavable linker and the second is a non-cleavable linker. This arrangement is described herein as 'Structure D' (FIG. 3D). In some embodiments, the first polypeptide chain comprises:

N' **TM1- cleavable L1-MM** C'

and the second polypeptide chain comprises:

N' **TM2- non-cleavable L2-C** C'

where TM1 and TM2 are the targeting moieties, L1 is the first linker, MM is the masking moiety, L2 is the second linker, and C is the cytokine moiety.

### 4. CLEAVAGE PRODUCT

Provided herein is a cleavage product, preparable by proteolytic cleavage of the proteolytically cleavable linker in the polypeptide constructs as described anywhere herein.

Upon proteolytic cleavage of the cleavable linker at the cleavage site, a cleavage product is formed.

Also provided herein is a cleavage product capable of comprising an active therapeutic moiety, preparable by proteolytic cleavage of the proteolytically cleavable linker in polypeptide drug construct as described anywhere herein. Where said polypeptide drug construct is a masked cytokine as described anywhere herein, upon proteolytic cleavage, a cleavage product comprising the cytokine moiety is formed. The cytokine moiety in the cleavage product is activated since it is no longer masked by the masking moiety. The cytokine moiety in the cleavage product is therefore capable of binding to the target protein.

Provided herein is a cleavage product of a 'heterodimeric' masked cytokine described anywhere herein.

Provided herein is a cleavage product of a 'linear' masked cytokine described anywhere herein.

Provided herein is a cleavage product of a "targeted' cytokine described anywhere herein.

The tumor cell environment is complex and can comprise multiple different proteases. As such, the precise site at which a given cleavable peptide within a polypeptide construct will be cleaved in the tumor cell environment may vary between tumor types, between patients with the same tumor type and even between cleavage products formed in the same tumor. Moreover, even after cleavage, further modification of the initial cleavage product, e.g. by removal of one or two terminal amino acids, may occur by the further action of proteases in the tumor cell environment. A distribution of cleavage products can thus be expected to form in the tumor cell environment of a patient following administration of a polypeptide construct as described herein.

It will be understood that a cleavage site as referred to herein refers to a site between two specific amino acid residues within the cleavable peptide that are a target for a protease known to be associated with a tumor cell environment. In this sense, there may be more than one cleavage site present in a cleavable peptide as described herein where different proteases cleave the cleavable peptide at different cleavage sites. It is also possible that more than one protease may act on the same cleavage site within a cleavable peptide. Discussion of protease cleavage sites can be found in the art.

Thus, the cleavable peptides disclosed herein may be cleaved by one or more proteases.

Provided herein is a cleavage product comprising a cytokine moiety capable of binding to it cognate receptor, preparable by proteolytic cleavage of the proteolytically cleavable linker in a masked cytokine as described anywhere herein.

Also provided herein is a distribution of cleavage products obtained or obtainable from a single structure of a masked cytokine, where each cleavage product within the distribution of cleavage products (i) is capable of binding to the target protein and (ii) comprises a cytokine (e.g., IL-15 cytokine) moiety as defined anywhere herein.

The location of the cleavable peptide in the polypeptide construct determines the structure of the resulting cleavage product.

It will be understood that the cleavage product may comprise a portion of the proteolytically cleavable peptide.

A "portion of a proteolytically cleavable peptide", refers to a part of the original proteolytically cleavable peptide sequence after cleavage at the cleavage site has occurred. After cleavage, further modification of the initial cleavage product, e.g. by removal of one or two terminal amino acids, may also occur by the further action of e.g. proteases in the tumor cell environment. As such, cleavage products within the distribution of cleavage products that might be formed might not contain any portion of the proteolytically cleavable peptide.

In some embodiments, a "portion" refers to 1 amino acid, 2 amino acids, 3 amino acids, 4 amino acids, 5 amino acids or 6 amino acids of the original proteolytically cleavable peptide sequence. In some embodiments, a "portion" refers to 2 amino acids of the original proteolytically cleavable peptide sequence. In some embodiments, a "portion" refers to 3 amino acids of the original proteolytically cleavable peptide sequence. In some embodiments, a "portion" refers to 4 amino acids of the original proteolytically cleavable peptide sequence.

In some embodiments, the 'portion' of the proteolytically cleavable peptide is from 3 to 6 amino acids in length. In some embodiments, the 'portion' of the proteolytically cleavable peptide is 3 or 4 amino acids in length.

Exemplary cleavage sites for cleavable linkers disclosed herein are disclosed herein **PVSL*RSGS** (SEQ ID NO:1) and **GMPKDLYHA*S** (SEQ ID NO:2) (* indicates a known or observed protease cleavage site within the cleavable peptide).

Accordingly, herein disclosed is the cleavage product of any one of the polypeptide constructs disclosed herein.

### 5. COMPOSITIONS

In some aspects, also provided herein are compositions comprising any of the polypeptide constructs described herein. In some embodiments, the composition comprises any of the drug constructs described herein. In some embodiments, the composition comprises any of the masked drug constructs described herein. In some embodiments, the composition comprises any of the masked cytokines described herein.

In some embodiments, the composition is a pharmaceutical composition.

In some embodiments, the composition comprises a polypeptide construct as described herein and further comprises one or more components. For example, in some embodiments, the composition comprises one or more pharmaceutically acceptable carriers, excipients, stabilizers, buffers, preservatives, tonicity agents, non-ionic surfactants or detergents, or other therapeutic agents or active compounds, or combinations thereof. The various embodiments of the composition are sometimes referred to herein as formulations.

### 6. METHODS OF TREATMENT

Provided herein are methods for treating or preventing a disease in a subject comprising administering to the subject an effective amount of any drug construct described herein, for example any masked cytokine, or compositions thereof. Said methods are not part of the claimed invention. In some embodiments, methods are provided for treating or preventing a disease in a subject comprising administering to the subject any composition described herein. Said methods are not part of the claimed invention. In some embodiments, the subject (e.g., a human patient) has been diagnosed with cancer or is at risk of developing such a disorder. In some embodiments, methods are provided for treating or preventing disease in a subject comprising administering to the subject an effective amount of any masked drug construct described herein or compositions thereof, wherein the masked drug construct is activated upon cleavage by an enzyme. Said methods are not part of the claimed invention. In some embodiments, the masked drug construct is activated at a tumor microenvironment. The masked drug construct is therapeutically active after it has cleaved. Thus, in some embodiments, the active agent is the cleavage product.

For the prevention or treatment of disease, the appropriate dosage of an active agent will depend on the type of disease to be treated, as defined herein, the severity and course of the disease, whether the agent is administered for preventive or therapeutic purposes, previous therapy, the subject's clinical history and response to the agent, and the discretion of the attending physician. The agent is suitably administered to the subject at one time or over a series of treatments.

In some embodiments, the protease acting to cleave the proteolytically cleavable peptide is a MMP. In some embodiments, the MMP is selected from the group consisting of MMP-1, MMP-2, MMP-3, MMP-7, MMP-8, MMP-9, MMP-10, MMP-11, MMP-12, MMP-13, MMP-14, MMP-15, MMP-16, MMP-17, MMP-19, MMP-20, MMP-21, MMP-23A, MMP-23B, MMP-24, MMP-25, MMP-27, MMP-28. In some embodiments, the protease acting to cleave the proteolytically cleavable peptide is MMP-2. In some embodiments, the protease acting to cleave the proteolytically cleavable peptide is MMP-7. In some embodiments, the protease acting to cleave the proteolytically cleavable peptide is MMP-8. In some embodiments, the protease acting to cleave the proteolytically cleavable peptide is MMP-9. In some embodiments, the protease acting to cleave the proteolytically cleavable peptide is MMP-10. In some embodiments, the protease acting to cleave the proteolytically cleavable peptide is MMP-14.

In some embodiments, the protease acting to cleave the proteolytically cleavable peptide is cathepsin, e.g., Cathepsin B, cathepsin D, cathepsin F, cathepsin K, cathepsin L, cathepsin V, cathepsin S, and cathepsin W. In some embodiments, the protease acting to cleave the proteolytically cleavable peptide is Cathepsin B (CatB).

In some embodiments, provided herein is a method of treatment or prevention of a cancer by administration of any masked drug construct, for example masked cytokine, or composition described herein in combination with an anticancer therapeutic protein. Said methods are not part of the claimed invention. The anti-cancer therapeutic protein can be any therapeutic protein capable of reducing cancer growth, interfering with cancer cell replication, directly or indirectly killing cancer cells, reducing metastasis, reducing tumor blood supply, or reducing cell survival. Exemplary anti-cancer therapeutic proteins may come in the form of an antibody or fragment thereof, an antibody derivative, a bispecific antibody, a chimeric antigen receptor (CAR) T cell, a fusion protein, or a bispecific T-cell engager (BiTE). In some embodiments, provided herein is a method of treatment or prevention of a cancer by administration of any masked cytokine or composition described herein in combination with CAR-NK (Natural Killer) cells. Said methods are not part of the claimed invention.

### 7. ARTICLES OF MANUFACTURE OR KITS

In another aspect, an article of manufacture or kit is provided which comprises any polypeptide construct described herein. The article of manufacture or kit may further comprise instructions for use of the polypeptides in the methods of the invention, for example instructions for use of masked cytokines in the method of the invention. Thus, in certain embodiments, the article of manufacture or kit comprises instructions for the use of a masked cytokine in methods for treating or preventing a disorder (e.g., a cancer) in an individual comprising administering to the individual an effective amount of a masked cytokine. For example, in certain embodiments, the article of manufacture or kit comprises instructions for the use of a masked polypeptide in methods for treating or preventing a disorder (e.g., a cancer) in an individual comprising administering to the individual an effective amount of a masked polypeptide. In certain embodiments, the individual is a human. In some embodiments, the individual has a disease selected from the group consisting of include leukemia, lymphoma, head and neck cancer, colorectal cancer, prostate cancer, pancreatic cancer, melanoma, breast cancer, neuroblastoma, lung cancer, ovarian cancer, osteosarcoma, bladder cancer, cervical cancer, liver cancer, kidney cancer, skin cancer or testicular cancer.

The article of manufacture or kit may further comprise a container. The container holds the formulation. In some embodiments, the formulation is a lyophilized formulation. In some embodiments, the formulation is a frozen formulation. In some embodiments, the formulation is a liquid formulation.

The article of manufacture or kit may further comprise a label or a package insert, which is on or associated with the container, may indicate directions for reconstitution and/or use of the formulation.

The article of manufacture or kit herein optionally further comprises a container comprising a second medicament, wherein the masked drug construct is a first medicament, and which article or kit further comprises instructions on the label or package insert for treating the subject with the second medicament, in an effective amount.

In another embodiment, provided herein is an article of manufacture or kit comprising the formulations described herein for administration in an auto-injector device.

### 8. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

As used in this specification and the appended claims, the singular forms "a," "an," and "the" include plural referents unless the content clearly dictates otherwise.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of' a aspects and embodiments.

As used herein, the term "and/or" refers to any one of the items, any combination of the items, or all of the items with which the term is associated. For instance, the phrase "A, B, and/or C" is intended to encompass each of the following embodiments: A, B, and C; A, B, or C; A or B; A or C; B or C; A and B; A and C; B and C; A and B or C; B and A or C; C and A or B; A (alone); B (alone); and C (alone).

The term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, as well as antibody fragments (e.g., Fab, F(ab')2, and Fv). The term "immunoglobulin" (Ig) is used interchangeably with "antibody" herein.

The terms "full-length antibody," "intact antibody" or "whole antibody" are used interchangeably to refer to an antibody in its substantially intact form, as opposed to an antibody fragment. Specifically, whole antibodies include those with heavy and light chains including an Fc region. The constant domains may be native sequence constant domains (e.g., human native sequence constant domains) or amino acid sequence variants thereof. In some cases, the intact antibody may have one or more effector functions.

An "antibody fragment" comprises a portion of an intact antibody, such as the antigen binding region and/or the variable region of the intact antibody, and/or the constant region of the intact antibody. Examples of an antibody fragment include the Fc region of the antibody, a portion of the Fc region, or a portion of the antibody comprising the Fc region. Examples of antigen-binding antibody fragments include domain antibodies (dAbs), Fab, Fab', F(ab')2 and Fv fragments; diabodies; linear antibodies (see U.S. Pat. No. 5,641,870, Example 2; Zapata et ah, Protein Eng. 8(10): 1057-1062 [1995]); single-chain antibody molecules, and multispecific antibodies formed from antibody fragments. Single heavy chain antibodies or single light chain antibodies can be engineered, or in the case of the heavy chain, can be isolated from camelids, shark, libraries or mice engineered to produce single heavy chain molecules.

The term "pharmaceutical formulation" refers to a preparation that is in such form as to permit the biological activity of the active ingredient to be effective, and that contains no additional components that are unacceptably toxic to a subject to which the formulation would be administered. Such formulations are sterile.

"Carriers" as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{™}, polyethylene glycol (PEG), and PLURONICS^{™}.

As used herein, the term "treatment" refers to clinical intervention designed to alter the natural course of the individual or cell being treated during the course of clinical pathology. Desirable effects of treatment include decreasing the rate of disease progression, ameliorating or palliating the disease state, and remission or improved prognosis. An individual is successfully "treated", for example, if one or more symptoms associated with a disorder (e.g., a neoplastic disease) are mitigated or eliminated. For example, an individual is successfully "treated" if treatment results in increasing the quality of life of those suffering from a disease, decreasing the dose of other medications required for treating the disease, reducing the frequency of recurrence of the disease, lessening severity of the disease, delaying the development or progression of the disease, and/or prolonging survival of individuals.

As used herein, "in conjunction with" or "in combination with" refers to administration of one treatment modality in addition to another treatment modality. As such, "in conjunction with" or "in combination with" refers to administration of one treatment modality before, during or after administration of the other treatment modality to the individual.

As used herein, the term "prevention" includes providing prophylaxis with respect to occurrence or recurrence of a disease in an individual. An individual may be predisposed to, susceptible to a disorder, or at risk of developing a disorder, but has not yet been diagnosed with the disorder. In some embodiments, masked cytokines described herein are used to delay development of a disorder.

As used herein, an individual "at risk" of developing a disorder may or may not have detectable disease or symptoms of disease, and may or may not have displayed detectable disease or symptoms of disease prior to the treatment methods described herein. "At risk" denotes that an individual has one or more risk factors, which are measurable parameters that correlate with development of the disease, as known in the art. An individual having one or more of these risk factors has a higher probability of developing the disorder than an individual without one or more of these risk factors.

An "effective amount" refers to at least an amount effective, at dosages and for periods of time necessary, to achieve the desired or indicated effect, including a therapeutic or prophylactic result.

An effective amount can be provided in one or more administrations. A "therapeutically effective amount" is at least the minimum concentration required to effect a measurable improvement of a particular disorder. A therapeutically effective amount herein may vary according to factors such as the disease state, age, sex, and weight of the patient, and the ability of the antibody to elicit a desired response in the individual. A therapeutically effective amount may also be one in which any toxic or detrimental effects of the masked cytokine are outweighed by the therapeutically beneficial effects. A "prophylactically effective amount" refers to an amount effective, at the dosages and for periods of time necessary, to achieve the desired prophylactic result. Typically, but not necessarily, since a prophylactic dose is used in subjects prior to or at the earlier stage of disease, the prophylactically effective amount can be less than the therapeutically effective amount.

"Chronic" administration refers to administration of the medicament(s) in a continuous as opposed to acute mode, so as to main the initial therapeutic effect (activity) for an extended period of time. "Intermittent" administration is treatment that is not consecutively done without interruption, but rather is cyclic in nature.

As used herein, an "individual" or a "subject" is a mammal. A "mammal" for purposes of treatment includes humans, domestic and farm animals, and zoo, sports, or pet animals, such as dogs, horses, rabbits, cattle, pigs, hamsters, gerbils, mice, ferrets, rats, cats, etc. In some embodiments, the individual or subject is human.

### EXAMPLES

The invention will be more fully understood by reference to the following examples.

Although some examples describe the engineering, production, and/or testing of "masked" versions of an polypeptide construct, some examples also employ parental "non-masked" versions of the polypeptide construct, such as for comparison, or other constructs that include one or more of the components described herein that are tested as controls for comparison. Accordingly, the description of, for instance, testing done on masked polypeptide constructs does not necessarily mean that non-masked versions of the construct were not also tested.

### Example 1: Cleavage of peptides by NAT vs. RCC culture supernatant

Sequences comprising cleavage peptides PVSLRSGS (SEQ ID NO:1) or GMPKDLYHAS (SEQ ID NO:2) (reference example) (shown in bold below) were incubated in either 'NAT' (Normal Adjacent Tissue) or 'RCC' (Renal Cell Carcinoma) culture supernatants, to test the specificity of each peptide's cleavage. Carfilzomib (CFZ) is also included in the incubation media as an inhibitor of proteosome, to inhibit false positive cleavage.

To this end, peptide sequencing by mass spectrometry was used to identify cleaved fragments produced for the synthetic peptides shown in the table below, using a published technique called multiplexed substrate profiling by mass spectrometry (MSP-MS) (O'Donoghue A.J. et al. Nat Methods. 2012 Nov;9(11):1095-100.) Cleavages were monitored in these reactions over time, and the peptides found to be cleaved in the earliest time points were deemed to be most sensitive to proteolytic activity in the conditioned media samples. Results are as follows:

| Substrate | Synthetic Tissue Sequence (bolded sequences show the cleavable peptide; * indicates cleavage site) | NAT + CFZ | RCC + CFZ | Earliest cleaved time point - NAT | Earliest cleaved time point - RCC |
|---|---|---|---|---|---|
| Alu-XT-019 | RVG**PVSL*R*SGS**GKI (SEQ ID NO: 9) | 1/5 | 5/5 | 5min | 15min |
| Alu-XT-091 | RQG**GM*PKDLYHA*S**AKQ (SEQ ID NO: 10) (reference example) | 1/5 | 5/5 | 5min | 15min |

### Example 2: Heterodimeric masked cytokines and targeted cytokines

This example demonstrates heterodimeric masked cytokines designed to incorporate a proteolytically cleavable peptide (CP) consisting of the amino acid sequence PVSLRSGS (SEQ ID NO: 1) or GMPKDLYHAS (SEQ ID NO:2) (reference example), or RPLALWRS (SEQ ID NO: 3) (reference example), or TQKPLGLS (SEQ ID NO:4) (reference example), or APAGLIVPYN (SEQ ID NO:5) (reference example), or PANLVAPDP (SEQ ID NO:6) (reference example), or IVGRPRHQGV (SEQ ID NO:7) (reference example), or RSKYLATA (SEQ ID NO:8) (reference example). Heterodimeric masked cytokines described in this example include a cleavable peptide on one polypeptide chain. Specifically masked cytokines with the following configurations are generated:

**Table 1: Masked cytokines with cleavable linkers**

| | First Polypeptide Chain | Second Polypeptide Chain | Cleavage Position |
|---|---|---|---|
| Structure A (Figure 3A) | N' HL1-non-cleavable L1-MM C' | N' HL2-cleavable L2-C C' | Cleavage Site (PVSLRSGS or GMPKDLYHAS, or RPLALWRS, or TQKPLGLS, or APAGLIVPYN, or PANLVAPDP, or IVGRPRHQGV, or RSKYLATA) on Cytokine arm |
| Structure B (Figure 3B) | N' HL1- cleavable L1-MM C' | N' HL2- non-cleavable L2-C C' | Cleavage Site (PVSLRSGS or GMPKDLYHAS, , or RPLALWRS, or TQKPLGLS, or APAGLIVPYN, or PANLVAPDP, or IVGRPRHQGV, or RSKYLATA) on masking arm |
| Structure C (Figure 3C) | N' TM1-HL1-cleavable L1-MM C' | N' TM2-HL2-non-cleavable L2-C C' | Cleavage Site (PVSLRSGS or GMPKDLYHAS, , or RPLALWRS, or TQKPLGLS, or APAGLIVPYN, or PANLVAPDP, or IVGRPRHQGV, or RSKYLATA) on masking arm |
| Structure D (Figure 3D) | N' TM1-HL1-non-cleavable L1-MM C' | N' TM2-HL2-cleavable L2-C C' | Cleavage Site (PVSLRSGS or GMPKDLYHAS, , or RPLALWRS, or TQKPLGLS, or APAGLIVPYN, or PANLVAPDP, or IVGRPRHQGV, or RSKYLATA) on cytokine arm |

HL1 is the first half life extension domain (e.g., Fc), L1 is the first linker, MM is the masking moiety (e.g., a cognate receptor for the cytokine such as CD122), HL2 is the second half life extension domain (e.g. Fc), L2 is the second linker, and C is the cytokine moiety (e.g., IL-15). TM is a targeting moiety (e.g., an antibody or an antigen binding domain thereof). Cleavage capacity of the heterodimeric constructs described above that incorporate PVSLRSGS or GMPKDLYHAS cleavage peptides are incubated with MMP7, 9 and 10 and analysed by SDS-PAGE. Cleavage is confirmed by HEK-Blue IL-2 bioassay. Briefly, constructs are diluted to 1uM with PBS. Recombinant cytokine (e.g., IL-15) at 0.1 mg/ml is used as a control. HEK-Blue cells are seeded with 50K cells/well at 150 uL per well in a 96 well plate. Constructs or recombinant cytokine are incubated at 37°C in 5% CO2 for 24 hours. 180uL QUANTI-blue (QB) solution and 20uL cell supernatant is incubated at 37°C for 1 hour. Results are read at 625nM on a plate reader to determine specific EC50.

### Example 3: Identification of new cleavable peptides

This example illustrates the discovery of 7 protease substrates, which are more cleaved by tumor than by plasma from RCC, and Head and Neck (H&N) cancer patients, and highly cleaved by recombinant MMP or Cathepsin B.

**Table 2: New protease substrates**

| **Peptide sequence** | **SEQ ID NO** |
|---|---|
| PVSLRSGS | SEQ ID NO:1 |
| RPLALWRS | SEQ ID NO:3 (reference example) |
| TQKPLGLS | SEQ ID NO:4 (reference example) |
| APAGLIVPYN | SEQ ID NO:5 (reference example) |
| PANLVAPDP | SEQ ID NO:6 (reference example) |
| **IVGRPRHQGV** | SEQ ID NO:7 (reference example) |
| **RSKYLATA** | SEQ ID NO:8 (reference example) |

In this example, the peptides library was subjected to cleavage screening by biological matrices, i.e., tumor conditioned media (TCM). The cleavage of the peptides library by plasma was performed for comparison. Each peptide in the library includes a fluorescent mark at one end of its sequence and a quencher on the other end of the sequence. Upon cleavage of the peptide, the quencher is separated from the fluorescent mark, thereby releasing the fluorescent mark. The peptide substrates with the highest ratio of (initial rate of cleavage by tumor conditioned media (TCM) - initial rate of cleavage by plasma) in pmol/min, from cancer patients were identified.

**Table 3: Ratio of Initial rate of cleavage by tumor conditioned media (TCM) - initial rate of cleavage by plasma**

| **Peptide sequence** | **RCC TCM-Plasma** | **H&N TCM --Plasma** |
|---|---|---|
| RPLALWRS (SEQ ID NO: 3) (reference example) | 0.5 | 0.0 |
| TQKPLGLS (SEQ ID NO: 4) (reference example) | 0.2 | 0.2 |
| APAGLIVPYN (SEQ ID NO: 5) (reference example) | 0.2 | 0.1 |
| PVSLRSGS (SEQ ID NO: 1) | 0.2 | 0.0 |
| PANLVAPDP (SEQ ID NO: 6) (reference example) | 0.1 | 0.4 |
| IVGRPRHQGV (SEQ ID NO: 7) (reference example) | 0.6 | 0.1 |
| RSKYLATA (SEQ ID NO: 8) (reference example) | 0.3 | 0.0 |

The peptides with high ratio of initial rate of cleavage by tumor conditioned media (TCM) to initial rate of cleavage by plasma were further screened using recombinant proteases. The peptide substrates that have high initial rates (uM/h) of cleavage by recombinant proteases were identified (Table 4).

**Table 4: Rates of cleavage by recombinant proteases**

| **Peptide sequence** | **MMP-1** | **MMP-2** | **MMP-7** | **MMP-8** | **MMP-9** | **MMP-10** | **MMP-14** | **CatB** |
|---|---|---|---|---|---|---|---|---|
| RPLALWRS (SEQ ID NO: 3) (reference example) | 0.1 | 1.1 | 0.2 | 0.2 | 0.2 | 0.0 | 0.0 | 0.3 |
| TQKPLGLS (SEQ ID NO: 4) (reference example) | 0.0 | 11.8 | 1.8 | 0.1 | 0.3 | 0.0 | 0.1 | 0.3 |
| APAGLIVPYN (SEQ ID NO: 5) (reference example) | 0.0 | 29.6 | 2.4 | 0.5 | 1.8 | 1.6 | 2.1 | 1.0 |
| PVSLRSGS (SEQ ID NO: 1) | 0.0 | 1.9 | 0.2 | 0.1 | 6.5 | 0.0 | 0.0 | 0.0 |
| PANLVAPDP (SEQ ID NO: 6) (reference example) | 2.7 | 31.2 | 2.5 | 7.2 | 8.8 | 1.8 | 0.7 | 0.0 |
| IVGRPRHQGV (SEQ ID NO: 7) (reference example) | 0.0 | 0.0 | 0.0 | 0.0 | 0.0 | 0.0 | 0.0 | 1.9 |
| RSKYLATA (SEQ ID NO: 8) (reference example) | 0.0 | 0.0 | 0.0 | 0.0 | 0.0 | 0.0 | 0.0 | 1.9 |

## Claims

1. A masked cytokine comprising:
a) a first polypeptide chain comprising a masking moiety linked to a first half-life extension moiety via a first linker; and
b) a second polypeptide chain comprising a cytokine moiety linked to a second half-life extension moiety via a second linker,
wherein the first half-life extension moiety is associated with the second half-life extension moiety, and
wherein at least the first linker or the second linker is a proteolytically cleavable peptide linker comprising a proteolytically cleavable peptide (CP) consisting of the amino acid sequence PVSLRSGS (SEQ ID NO: 1).

2. The masked cytokine according to claim 1, wherein the second linker is the proteolytically cleavable linker and the first linker is a non-cleavable linker.

3. The masked cytokine according to claim 1, wherein the first linker is the proteolytically cleavable linker and the second linker is a non-cleavable linker.

4. A targeted cytokine comprising:
a) a targeting moiety;
b) a cytokine moiety;
c) a masking moiety; and
d) an Fc domain comprising a first Fc polypeptide linked to the cytokine moiety through a first linker and a second Fc polypeptide linked to the masking moiety through a second linker, wherein the first or the second linker is a cleavable linker such that the masking moiety releases the cytokine moiety upon cleavage;
wherein the targeting moiety is linked to the Fc domain through one or both of the first and second Fc polypeptides; and
wherein at least the first linker or the second linker is a proteolytically cleavable peptide linker comprising a proteolytically cleavable peptide (CP) consisting of the amino acid sequence PVSLRSGS (SEQ ID NO: 1).

5. The targeted cytokine of claim 4, wherein the second linker is the proteolytically cleavable linker and the first linker is a non-cleavable linker.

6. The targeted cytokine of claim 4, wherein the first linker is the proteolytically cleavable linker and the second linker is a non-cleavable linker.

7. The targeted cytokine according to any one of claims 4 to 6, wherein the targeting moiety is an antibody or an antigen binding domain thereof.

8. A nucleic acid encoding the masked cytokine according to any one of claims 1 to 3, or the targeted cytokine according to any one of claims 4 to 7.

9. A vector comprising a nucleic acid according to claim 8.

10. A host cell comprising a nucleic acid according to claim 8, or a vector according to claim 9.

11. A composition comprising the masked cytokine according to any one of claims 1 to 3, or the targeted cytokine according to any one of claims 4 to 7.

12. A pharmaceutical composition comprising the masked cytokine according to claims 1 to 3, or the targeted cytokine according to any one of claims 4 to 7, and a pharmaceutically acceptable carrier.

13. A kit comprising the masked cytokine according to any of claims 1 to 3, or the targeted cytokine according to any one of claims 4 to 7, or the composition according to claim 11, or the pharmaceutical composition according to claim 12.

14. A masked cytokine according to any one of claims 1 to 3, or a targeted cytokine according to any one of claims 4 to 7, or a composition according to claim 11, or a pharmaceutical composition according to claim 12 for use in medicine.

15. A masked cytokine according to any one of claims 1 to 3, or a targeted cytokine according to any one of claims 4 to 7, or a composition according to claim 11, or a pharmaceutical composition according to claim 12 for use in treating or preventing cancer.

## Patentansprüche

1. Maskiertes Zytokin, umfassend:
a) eine erste Polypeptidkette, die einen maskierenden Anteil umfasst, der über einen ersten Linker mit einem ersten Halbwertszeitverlängerungsanteil verknüpft ist; und
b) eine zweite Polypeptidkette, die einen Zytokinanteil umfasst, der über einen zweiten Linker mit einem zweiten Halbwertszeitverlängerungsanteil verknüpft ist,
wobei der erste Halbwertszeitverlängerungsanteil dem zweiten Halbwertszeitverlängerungsanteil zugeordnet ist und
wobei mindestens der erste Linker oder der zweite Linker ein proteolytisch spaltbarer Peptid-Linker ist, der ein proteolytisch spaltbares Peptid (CP) umfasst, das aus der Aminosäuresequenz PVSLRSGS (SEQ ID NO: 1) besteht.

2. Maskiertes Zytokin nach Anspruch 1, wobei der zweite Linker der proteolytisch spaltbare Linker ist und der erste Linker ein nicht spaltbarer Linker ist.

3. Maskiertes Zytokin nach Anspruch 1, wobei der erste Linker der proteolytisch spaltbare Linker ist und der zweite Linker ein nicht spaltbarer Linker ist.

4. Gerichtetes Zytokin, umfassend:
a) einen abzielenden Anteil;
b) einen Zytokinanteil;
c) einen maskierenden Anteil; und
d) eine Fc-Domäne, die ein erstes Fc-Polypeptid, das über einen ersten Linker mit dem Zytokinanteil verknüpft ist, und ein zweites Fc-Polypeptid, das über einen zweiten Linker mit dem maskierenden Anteil verknüpft ist, umfasst, wobei der erste oder der zweite Linker ein spaltbarer Linker derart ist, dass der maskierende Anteil den Zytokinanteil bei Spaltung freisetzt;
wobei der abzielende Anteil über einen oder beide des ersten und des zweiten Fc-Polypeptids mit der Fc-Domäne verknüpft ist; und
wobei mindestens der erste Linker oder der zweite Linker ein proteolytisch spaltbarer Peptid-Linker ist, der ein proteolytisch spaltbares Peptid (CP) umfasst, das aus der Aminosäuresequenz PVSLRSGS (SEQ ID NO: 1) besteht.

5. Gerichtetes Zytokin nach Anspruch 4, wobei der zweite Linker der proteolytisch spaltbare Linker ist und der erste Linker ein nicht spaltbarer Linker ist.

6. Gerichtetes Zytokin nach Anspruch 4, wobei der erste Linker der proteolytisch spaltbare Linker ist und der zweite Linker ein nicht spaltbarer Linker ist.

7. Gerichtetes Zytokin nach einem der Ansprüche 4 bis 6, wobei der abzielende Anteil eine antikörper- oder antigenbindende Domäne dessen ist.

8. Nukleinsäure, die das maskierte Zytokin nach einem der Ansprüche 1 bis 3 oder das gerichtete Zytokin nach einem der Ansprüche 4 bis 7 codiert.

9. Vektor, umfassend eine Nukleinsäure nach Anspruch 8.

10. Wirtszelle, umfassend eine Nukleinsäure nach Anspruch 8 oder einen Vektor nach Anspruch 9.

11. Zusammensetzung, umfassend das maskierte Zytokin nach einem der Ansprüche 1 bis 3 oder das gerichtete Zytokin nach einem der Ansprüche 4 bis 7.

12. Pharmazeutische Zusammensetzung, umfassend das maskierte Zytokin nach den Ansprüchen 1 bis 3 oder das gerichtete Zytokin nach einem der Ansprüche 4 bis 7 und einen pharmazeutisch verträglichen Träger.

13. Kit, umfassend das maskierte Zytokin nach einem der Ansprüche 1 bis 3 oder das gerichtete Zytokin nach einem der Ansprüche 4 bis 7 oder die Zusammensetzung nach Anspruch 11 oder die pharmazeutische Zusammensetzung nach Anspruch 12.

14. Maskiertes Zytokin nach einem der Ansprüche 1 bis 3 oder gerichtetes Zytokin nach einem der Ansprüche 4 bis 7 oder Zusammensetzung nach Anspruch 11 oder pharmazeutische Zusammensetzung nach Anspruch 12 zur Verwendung in der Medizin.

15. Maskiertes Zytokin nach einem der Ansprüche 1 bis 3 oder gerichtetes Zytokin nach einem der Ansprüche 4 bis 7 oder Zusammensetzung nach Anspruch 11 oder pharmazeutische Zusammensetzung nach Anspruch 12 zur Verwendung in der Behandlung oder Verhinderung von Krebs.

## Revendications

1. Cytokine masquée comprenant :
a) une première chaîne polypeptidique comprenant un fragment de masquage lié à un premier fragment d'extension de demi-vie via un premier lieur ; et
b) une seconde chaîne polypeptidique comprenant un fragment de cytokine lié à un second fragment d'extension de demi-vie via un second lieur,
dans laquelle le premier fragment d'extension de demi-vie est associé au second fragment d'extension de demi-vie, et
dans laquelle au moins le premier ou le second lieur est un lieur peptidique clivable par protéolyse comprenant un peptide clivable par protéolyse (CP) constitué de la séquence d'acides aminés PVSLRSGS (SEQ ID NO : 1).

2. Cytokine masquée selon la revendication 1, dans laquelle le second lieur est le lieur clivable par protéolyse et le premier lieur est un lieur non clivable.

3. Cytokine masquée selon la revendication 1, dans laquelle le premier lieur est le lieur clivable par protéolyse et le second lieur est un lieur non clivable.

4. Cytokine ciblée comprenant :
**a)** un fragment de ciblage ;
**b)** un fragment de cytokine ;
**c)** un fragment de masquage ; et
**d)** un domaine Fc comprenant un premier polypeptide Fc lié au fragment cytokine par l'intermédiaire d'un premier lieur et un second polypeptide Fc lié au fragment de masquage par l'intermédiaire d'un second lieur, dans laquelle le premier ou le second lieur est un lieur clivable de sorte que le fragment de masquage libère le fragment de cytokine lors du clivage ;
dans laquelle le fragment de ciblage est lié au domaine Fc par l'intermédiaire d'un ou des deux premier et second polypeptides Fc ; et
dans laquelle au moins le premier ou le second lieur est un lieur peptidique clivable par protéolyse comprenant un peptide clivable par protéolyse (CP) constitué de la séquence d'acides aminés PVSLRSGS (SEQ ID NO : 1).

5. Cytokine ciblée selon la revendication 4, dans laquelle le second lieur est le lieur clivable protéolytiquement et le premier lieur est un lieur non clivable.

6. Cytokine ciblée selon la revendication 4, dans laquelle le premier lieur est le lieur clivable par protéolyse et le second lieur est un lieur non clivable.

7. Cytokine ciblée selon l'une quelconque des revendications 4 à 6, dans laquelle le fragment de ciblage est un anticorps ou un domaine de liaison à l'antigène de celui-ci.

8. Acide nucléique codant la cytokine masquée selon l'une quelconque des revendications 1 à 3, ou la cytokine ciblée selon l'une quelconque des revendications 4 à 7.

9. Vecteur comprenant un acide nucléique selon la revendication 8.

10. Cellule hôte comprenant un acide nucléique selon la revendication 8, ou un vecteur selon la revendication 9.

11. Composition comprenant la cytokine masquée selon l'une quelconque des revendications 1 à 3, ou la cytokine ciblée selon l'une quelconque des revendications 4 à 7.

12. Composition pharmaceutique comprenant la cytokine masquée selon les revendications 1 à 3, ou la cytokine ciblée selon l'une quelconque des revendications 4 à 7, et un support pharmaceutiquement acceptable.

13. Kit comprenant la cytokine masquée selon l'une quelconque des revendications 1 à 3, ou la cytokine ciblée selon l'une quelconque des revendications 4 à 7, ou la composition selon la revendication 11, ou la composition pharmaceutique selon la revendication 12.

14. Cytokine masquée selon l'une quelconque des revendications 1 à 3, ou cytokine ciblée selon l'une quelconque des revendications 4 à 7, ou composition selon la revendication 11, ou composition pharmaceutique selon la revendication 12 destinée à être utilisée en médecine.

15. Cytokine masquée selon l'une quelconque des revendications 1 à 3, ou une cytokine ciblée selon l'une quelconque des revendications 4 à 7, ou composition selon la revendication 11, ou composition pharmaceutique selon la revendication 12 destinée à être utilisée dans le traitement ou la prévention du cancer.
